# EUROPEAN PATENT SPECIFICATION

(11) **EP 4 289 359 B1**
(45) Date of publication and mention of the grant of the patent: **12.08.2026**
(21) Application number: 22748964.8
(22) Date of filing: 25.01.2022
(51) Int. Cl.: A61B 5/369, A61B 5/316

(54) **SELF-RESPONSIVE DETECTION PARAMETER OPTIMIZATION METHOD AND SYSTEM FOR IMPLANTABLE ELECTRICAL STIMULATION APPARATUS**
VERFAHREN UND SYSTEM ZUR OPTIMIERUNG VON SELBSTANSPRECHENDEN DETEKTIONSPARAMETERN FÜR IMPLANTIERBARE ELEKTRISCHE STIMULATIONSVORRICHTUNG
PROCÉDÉ ET SYSTÈME D'OPTIMISATION DE PARAMÈTRE DE DÉTECTION AUTO-RÉACTIF POUR APPAREIL DE STIMULATION ÉLECTRIQUE IMPLANTABLE

(30) Priority: 05.02.2021 CN 202110164481
(43) Date of publication of application: 13.12.2023
(73) Proprietor: Hangzhou Nuowei Medical Technology Co., Ltd, Hangzhou, Zhejiang 311100 (CN)
(72) Inventor: CAO, Peng, Hangzhou, Zhejiang 311100 (CN); LIN, Ting, Hangzhou, Zhejiang 311100 (CN); WU, Chenghan, Hangzhou, Zhejiang 311100 (CN); CHEN, Xinlei, Hangzhou, Zhejiang 311100 (CN)
(74) Representative: Boult Wade Tennant LLP
(86) International application number: PCT/CN2022/073771
(87) International publication number: WO 2022/166685

(56) References cited:
- CN-A- 109 009 085
- CN-A- 109 009 085
- CN-A- 109 475 317
- CN-A- 109 475 317
- CN-A- 112 842 362
- US-A- 6 016 449
- US-A1- 2003 004 428
- US-A1- 2003 004 428
- US-A1- 2006 235 489
- US-A1- 2008 161 713
- US-A1- 2008 161 713
- US-A1- 2008 195 166
- US-A1- 2008 195 166
- US-A1- 2008 319 335
- US-A1- 2008 319 335
- US-A1- 2011 270 096
- US-A1- 2011 270 096

## Description

### Technical Field

The present disclosure relates to the field of medical device technology, and more particularly, to a self-responsive detection parameter optimization method and system for an implantable electrical stimulation device.

### Background

Currently, implantable medical systems have been widely applied in clinical medicine, including implantable electrical stimulation systems, implantable drug injection systems, etc. The implantable electrical stimulation systems include implantable neural electrical stimulation systems and implantable cardiac electrical stimulation systems. The implantable neural electrical stimulation system primarily comprises an implantable electrical pulse generator implanted in the body, stimulation electrodes, and an external controller. The electrical stimulation pulses generated by the implantable electrical pulse generator are transmitted to the stimulation electrodes, which then transmit them to specific neural targets for electrical stimulation, thereby treating conditions such as Parkinson's and epilepsy.

Typically, while the implantable electrical pulse generator is using electrical stimulation on neural targets to treat conditions, it also collects sample signals via a collection circuit to further determine the treatment effect of the electrical stimulation system on the conditions based on the sample signals, thereby adjusting the electrical stimulation pulses sent from the implantable electrical pulse generator to the stimulation electrodes. However, there is no way to predict and intervene in advance to prevent the occurrence of events such as Parkinson's and epilepsy. How to predict conditions and intervene in advance based on the prediction results is a technical problem that urgently needs to be solved.

D1 (US 2008/319335 A1) discloses methods and systems for detecting neurological events and approximating a target detection rate. A target detection rate may be identified for the neurological event. Electrographic signals incident on a neurological event detector may be monitored. Each signal may be compared to a threshold value for a parameter. As the threshold value varies, it has a predictable effect on a detection rate of the neurological event. A rate at which the electrographic signals exceed the threshold value may be measured and compared to the target detection rate. The threshold value may be adjusted to minimize the difference between the measured detection rate and the target detection rate. However, D1 is silent on how to set a detection flag to determine the current detection parameters. US 2003/004428 also discloses known methods and systems for detecting neurological events.

### Summary

The purpose of one or more embodiments of this specification is to provide a self-responsive detection parameter optimization method and system for an implantable electrical stimulation device. This can perform real-time detection and prediction of conditions, and therefore can intervene in advance based on prediction results, preventing events such as epilepsy.

To solve the above technical problems, one or more embodiments of this specification are implemented as follows:
In a first aspect, a self-responsive detection parameter optimization method for an implantable electrical stimulation device is proposed, which comprises: collecting sample signals based on current detection parameters; determining that the sample signal exceeds a preset detection threshold; self-responsively obtaining optimal detection parameters; and adjusting the current detection parameters based on the optimal detection parameters.
In a second aspect, an implantable electrical stimulation device is proposed, which comprises: a collecting module for collecting sample signals based on current detection parameters; a determining module for determining whether the sample signal exceeds a preset detection threshold; an obtaining module for self-responsively obtaining optimal detection parameters; and an adjusting module for adjusting the current detection parameters based on the optimal detection parameters.
In a third aspect, an implantable electrical stimulation system is proposed, which comprises an external device and the implantable electrical stimulation device as mentioned above. The external device is communicatively connected with the implantable electrical stimulation device.
In a fourth aspect, a storage medium for computer-readable storage is proposed. The storage medium stores one or more programs. When the one or more programs are executed by one or more processors, the steps of the self-responsive detection parameter optimization method mentioned above are implemented.

From the technical solutions provided by one or more embodiments of this specification, it can be seen that the self-responsive detection parameter optimization method for implantable electrical stimulation devices provided by this application first collects sample signals based on current detection parameters. After determining that the sample signal exceeds a preset detection threshold, the optimal detection parameters are obtained. The detection threshold here is a range value of the electroencephalogram (EEG) signal when determining whether conditions such as epilepsy are occurring or about to occur based on the collected sample signal, for example, the EEG signal. If the current detection parameters are set too small, it is possible to trigger a responsive treatment under normal circumstances, leading to overly high sensitivity of the responsive treatment. High sensitivity also increases the risk of side effects. Therefore, after determining that the sample signal exceeds the preset detection threshold, the optimal detection parameters are self-responsively obtained. The current detection parameters are adjusted based on the obtained optimal detection parameters, that is, the current detection parameters are adjusted to the optimal detection parameters. This further optimizes the current detection parameters, enabling precise detection of events such as epilepsy during real-time patient monitoring. This reduces the false detection rate while triggering responsive treatment, achieving the goal of precise treatment. As a result, interventions to prevent events such as epilepsy can be timely implemented.

### Brief Description of the Drawings

To more clearly illustrate the technical solutions of one or more embodiments or prior art set forth in this specification, the following provides a simple introduction to the diagrams used in the description of one or more embodiments or prior art. Obviously, the diagrams described below are just some of the embodiments recited in this specification. For those skilled in the art, other diagrams can be obtained based on these diagrams without exerting creative effort.
Fig.1 is a schematic diagram of the steps of a self-responsive detection parameter optimization method for an implantable electrical stimulation device provided by an embodiment of this specification.
Fig.2 is a schematic diagram of the steps of another self-responsive detection parameter optimization method for an implantable electrical stimulation device provided by an embodiment of this specification.
Fig.3 is a schematic diagram of the steps of yet another self-responsive detection parameter optimization method for an implantable electrical stimulation device provided by an embodiment of this specification.
Fig.4 is a schematic diagram of the steps of yet another self-responsive detection parameter optimization method for an implantable electrical stimulation device provided by an embodiment of this specification.
Fig.5 is a schematic diagram of the steps of yet another self-responsive detection parameter optimization method for an implantable electrical stimulation device provided by an embodiment of this specification.
Fig.6 is a schematic diagram of the steps of yet another self-responsive detection parameter optimization method for an implantable electrical stimulation device provided by an embodiment of this specification.
Fig.7 is a schematic diagram of the steps of yet another self-responsive detection parameter optimization method for an implantable electrical stimulation device provided by an embodiment of this specification.
Fig.8 is a schematic diagram of the structure of an implantable electrical stimulation device provided by an embodiment of this specification.
Fig.9 is a schematic diagram of the structure of another implantable electrical stimulation device provided by an embodiment of this specification.

### Detailed Description of the Examples

To allow those skilled in the art to better understand the technical solutions in this specification, the technical solutions in one or more embodiments in this specification will be clearly and completely described in connection with the accompanying drawings in one or more embodiments of this specification. Obviously, the described one or more embodiments are merely part of the embodiments in this specification, not all of them. Based on the one or more embodiments in this specification, all other embodiments obtained by those skilled in the art without making inventive efforts should fall within the scope of protection of this document.

The self-responsive detection parameter optimization method for implantable electrical stimulation devices provided by this application can conduct real-time detection and prediction of diseases, thus enabling intervention in advance based on the predicted results to prevent events such as epilepsy. The following will detail the self-responsive detection parameter optimization method for implantable electrical stimulation devices provided by this application and its various steps.

The implantable electrical stimulation device applicable to the self-responsive detection parameter optimization method for implantable electrical stimulation devices provided by this application can be implanted into the human body. It comes into contact with brain tissue through the sampling circuit, collects electroencephalogram signals of human brain tissue, and adjusts the stimulation circuit after analyzing and determining the information displayed by the sample signal. The adjusted stimulation circuit sends electrical pulses to the brain tissue.

It should be noted that the sample signal mentioned in this application can be physiological signals collected from humans or animals.

### Embodiment 1

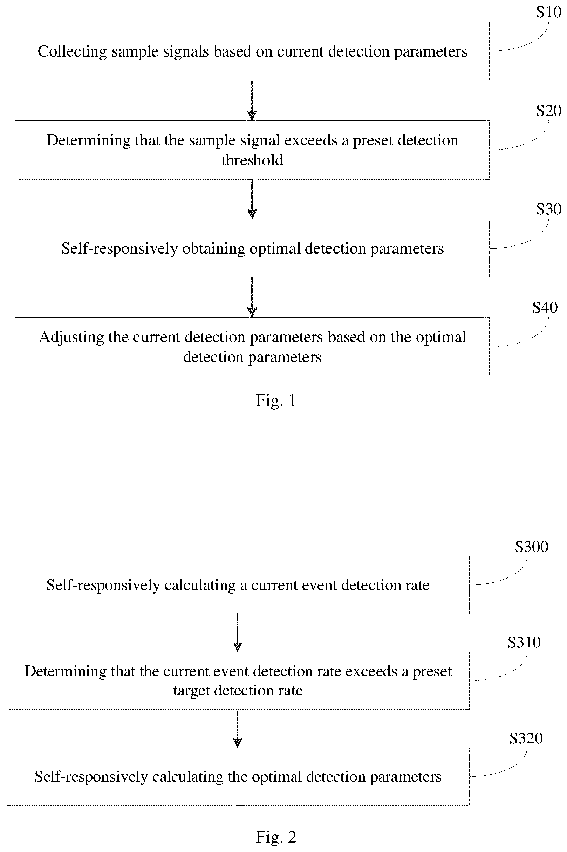

Referring to Fig.1, the embodiment of the specification provides a self-responsive detection parameter optimization method for implantable electrical stimulation devices, which is applicable to clinical medical implantable electrical stimulation systems. It can carry out real-time detection on patients with conditions such as epilepsy, and accurately detect the occurrence of events such as epilepsy during the treatment process, so as to intervene in treatment early and avoid the occurrence of events such as epilepsy. This self-responsive detection parameter optimization method for implantable electrical stimulation devices continuously obtains the optimal detection parameters in the process of real-time detection, thereby collecting more accurate sample signals based on the optimal detection parameters. The self-responsive detection parameter optimization method for implantable electrical stimulation devices provided by the embodiment of this specification comprises:
Step 10: collecting sample signals based on current detection parameters

The first thing an implantable electrical stimulation device needs to do is to collect sample signals such as electroencephalogram signals within a patient's skull to get the electroencephalogram sample values. The sample signals can also be Electrocardiograph ("ECG") signals, nerve electrical signals, etc., depending on the implantable electrical stimulation device. The current detection parameters can be initially set by medical staff based on preliminary determination of the patient's condition, generally including detection algorithms, detection thresholds, detection intervals, etc. The current detection parameters can also be factory default values of the detection device.
Step 20: determining that the sample signal exceeds a preset detection threshold

After obtaining the sample signal, process it and compare the sample signal with the preset detection threshold to determine whether to optimize the current detection parameters. The setting principle of the preset detection threshold can be determined based on the detection accuracy and sensitivity of events such as epilepsy. The preset detection threshold can be set by medical staff, for example, it can be set accordingly based on the parameters contained in the sample signal. When the sample signal contains multiple parameters, the corresponding preset detection threshold can contain multiple values. If the sample signal exceeds one of the preset detection thresholds, it is determined that the sample signal exceeds the preset detection threshold. For example, the preset detection threshold can be 50% of the sample signal when events like epilepsy occur.
Step 30: self-responsively obtaining optimal detection parameters

After determining that the sample signal exceeds the preset detection threshold, the optimal detection parameters are self-responsively obtained with the aim of replacing the current detection parameters with the optimal ones.

The optimal detection parameters can be detection parameters that have been preset based on clinical experience, or they can be detection parameters obtained in other ways; no specific limitation is provided here.
Step 40: adjusting the current detection parameters based on the optimal detection parameters

Based on the obtained optimal detection parameters, the current detection parameters are adjusted. The microprocessor of the implantable electrical stimulation device can send a parameter adjustment instruction and the optimal detection parameters to the sampling circuit. After receiving the parameter adjustment instruction, the sampling circuit executes the parameter adjustment instruction, replacing the current detection parameters with the optimal ones to become the next set of detection parameters for collecting sample signals.

Referring to Fig. 2, in some embodiments, the self-responsive detection parameter optimization method for implantable electrical stimulation devices provided by the embodiment of this specification comprises, in Step 30, the self-responsively obtaining the optimal detection parameters, specifically including:
Step 300: self-responsively calculating a current event detection rate

In step 30 of self-responsively obtaining the optimal detection parameters, it can be obtained through the self-responsive detection parameter optimization method provided by the embodiment of this specification. After determining that the sample signal exceeds the preset detection threshold and confirming the detection of events such as epilepsy, the current event detection rate is then self-responsively calculated. The current event detection rate is the detection rate of events such as epilepsy up to now. The calculation of the detection rate can be the number of times events such as epilepsy have been detected from the start of the detection to now, divided by the total number of detections. The total number of detections in this set time period during the real-time detection process can be known, and the number of times events such as epilepsy are detected can also be known, so the current event detection rate can be self-responsively calculated.
Step 310: determining that the current event detection rate exceeds a preset target detection rate

The preset target detection rate can be set based on actual clinical experience, for example, the preset target detection rate is 80% for a week. It is determined whether the current event detection rate exceeds the preset target detection rate. After determining that the current event detection rate is higher than the preset target detection rate, it can be inferred that the current detection parameters for events such as epilepsy are not accurate and sensitive enough, and it is necessary to further optimize the current detection parameters in order to detect events such as epilepsy more timely and accurately.
Step 320: self-responsively calculating the optimal detection parameters

The optimal detection parameters are self-responsively calculated. After self-responsively obtaining the optimal detection parameters, the implantable electrical stimulation device sends the optimal detection parameters and parameter adjustment instructions to the collecting circuit, and the collecting circuit adjusts the original current detection parameters based on the optimal detection parameters.

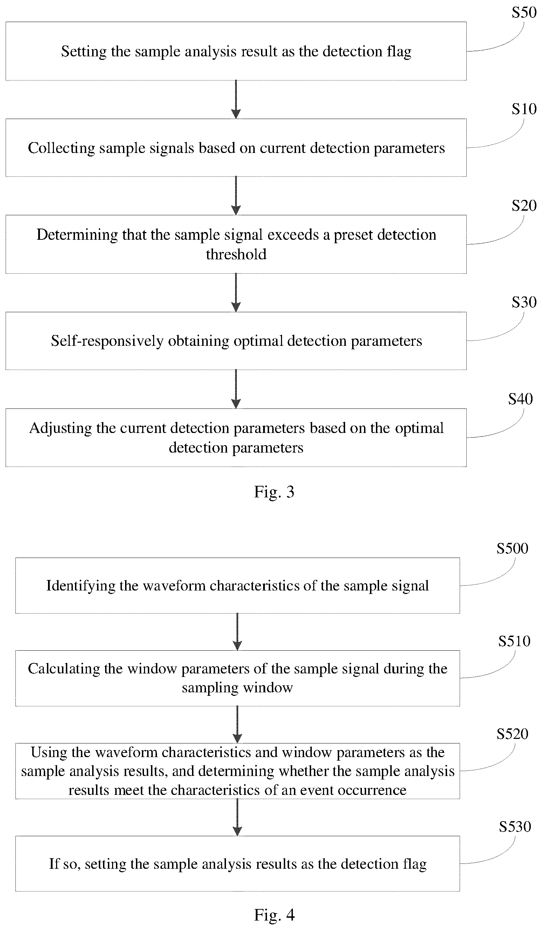

Referring to Fig. 3, in some embodiments, before Step 10: collecting sample signals based on current detection parameters, the self-responsive detection parameter optimization method for implantable electrical stimulation devices provided by the embodiment of this specification also comprises:
Step 50: setting the sample analysis result as a detection flag

Before starting each detection cycle to collect sample signals, the sample analysis results can be set as detection flags. The sample analysis results here can be the recent sample analysis results of the patient compared to the current ones as historical data, for example, the sample results collected during the last sample window period. After analyzing the recent sample analysis results, the sample analysis results obtained can be used to determine the symptoms or physiological characteristics of the patient when events such as epilepsy occur, which can be quantified as waveform characteristics, and then set the sample analysis result as a detection flag. Sample analysis results include half-wave amplitude, half-wave duration, and other half-wave characteristics. Subsequent detection is carried out based on the detection flag. For example, different current detection parameters are used to collect sample signals. For different patients at different times, the recent sample analysis results are different, corresponding detection flags can be set to carry out corresponding detection, which improves the individualization and targeting of real-time detection for patients.

Referring to Fig. 4, in some embodiments, the self-responsive detection parameter optimization method for implantable electrical stimulation devices provided by the embodiment of this specification comprises step 50: setting the sample analysis result as the detection flag, which specifically comprises:
Step 500: identifying the waveform characteristics of the sample signal

The waveform characteristics of the sample signal can be identified by observing changes in the amplitude of the sample signal, such as identifying the half-wave characteristics of the sample signal, so that epileptic waves and normal EEG waves can be identified based on the half-wave characteristics. The sample signal is a recent sample analysis result, which could be the sample signal during the last sampling window.

The waveform characteristics of the sample signal are identified. If the waveform characteristics match the symptoms or physiological characteristics of the patient's epileptic events, these waveform characteristics are marked and stored to be used as detection flags for the next sampling window.
Step 510: calculating the window parameters of the sample signal during the sampling window

The sampling window period is the period when the patient is being monitored in real-time. The window parameters of the sample signal during the last sampling window, such as changes in sampling amplitude and frequency, can be calculated, and the line length function and area function of the last sampling window can be calculated. The window parameters of the sample signal during the last sampling window can be used as detection flags for the next sampling window.

Step 520: using the waveform characteristics and window parameters as the sample analysis results, and determining whether the sample analysis results meet the characteristics of an event occurrence.

The collected sample signals are compared with the preset detection threshold, and the current detection parameters can be automatically adjusted. Before this, feature recognition is performed on the collected electrical signals. Due to individual differences and complexity in the waveform features themselves, combining the recognized different features in different ways (such as the combination of line length and area, line length and bandpass, etc.), can improve the accuracy of real-time detection and prediction, increase the detection rate of EEG signals during the occurrence of events such as the epileptic events, and prevent the occurrence of epileptic events.

After obtaining the waveform characteristics, such as half-wave characteristics, and window parameters as recent sample analysis results, whether the waveform characteristics and window parameters is further determined to meet the symptoms and physiological characteristics of event occurrence. If they do, set these sample analysis results as detection flags. After the detection flag is set, corresponding detection operations are performed based on the detection flag.
Step 530: if so, setting the sample analysis results as the detection flag

Whether the waveform characteristics and window parameters of the sample signals during the recent sampling window period, which are used as sample analysis results, are the physiological characteristics of the patient's epileptic seizures is determined. If so, the sample analysis results are set as the detection flag, and then corresponding detection instructions are initiated, so that corresponding detection parameters can be used to collect the sample signals.

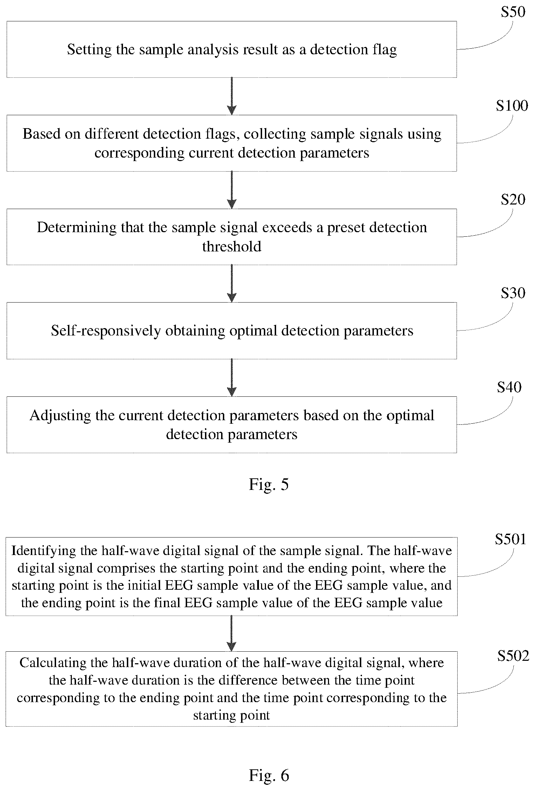

Referring to Fig. 5, in some embodiments, the self-responsive detection parameter optimization method for implantable electrical stimulation devices provided by the embodiment of this specification, after completing step 50: under the condition of setting the analysis results as the detection flag, step 10: collecting sample signals based on current detection parameters, specifically comprises:
Step 100: based on different detection flags, collecting sample signals using corresponding current detection parameters

Each patient has different detection flags at different periods, and the sample signals are collected using the corresponding current detection parameters. Different patients have different detection flags, and the sample signals are collected using different current detection parameters, thereby achieving personalized and targeted real-time detection for patients.

Referring to Fig. 6, in some embodiments, the self-responsive detection parameter optimization method for implantable electrical stimulation devices provided by the embodiment of this specification comprises step 500: identifying the waveform characteristics of the sample signal, which specifically comprises:
Step 501: identifying the half-wave digital signal of the sample signal. The half-wave digital signal comprises the starting point and the ending point, where the starting point is the initial EEG sample value of the EEG sample value, and the ending point is the final EEG sample value of the EEG sample value.

Identifying the waveform characteristics of the sample signal could be identifying the half-wave digital signal of the sample signal. The half-wave digital signal comprises the starting point and the ending point. The starting point of the sample signal during the sampling window period equals the initial EEG sample value during that sampling window period, and the ending point equals the final EEG sample value during that sampling window period.

Step 502: calculating the half-wave duration of the half-wave digital signal, where the half-wave duration is the difference between the time point corresponding to the ending point and the time point corresponding to the starting point.

The half-wave duration of the half-wave digital signal is calculated, which is equal to the difference between the time point corresponding to the ending point and the time point corresponding to the starting point.

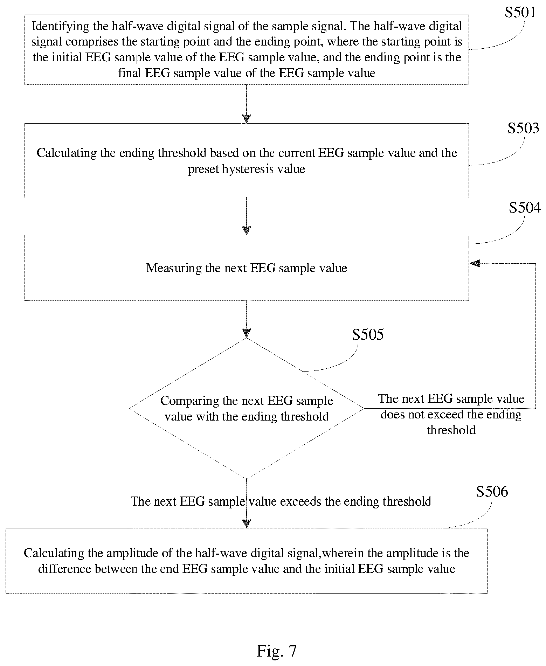

Referring to Fig. 7, in some embodiments, the self-responsive detection parameter optimization method for implantable electrical stimulation devices provided by the embodiment of this specification comprises step 501: identifying the half-wave digital signal of the sample signal, which comprises a starting point and an ending point, where the starting point is the initial EEG sample value of the EEG sample value, and the ending point is the final EEG sample value of the EEG sample value, which specifically comprises:
Step 503: calculating the ending threshold based on the current EEG sample value and the preset hysteresis value

The current EEG sample value referred to here is the EEG sample value during the current sampling window period, as mentioned above. The hysteresis value is preset and refers to the hysteresis value of the sample value. The preset hysteresis value is a fixed value and does not change with each sampling window period. The ending threshold is used to compare with the next EEG sample value to determine the end EEG sample value of the current EEG sample value.

Based on the current EEG sample value during the current sampling window period and the preset hysteresis value, the ending threshold is calculated.

The ending threshold is a range value obtained by adding or subtracting the hysteresis value from the initial EEG sample value of the current EEG sample value, and then measuring the current EEG sample value during the current sampling window period in turn, comparing the current EEG sample value with the ending threshold. If the current EEG sample value is less than the ending threshold, the ending threshold is recalculated, the current EEG sample value is measured, and the current EEG sample value is compared with the ending threshold until the current EEG sample value exceeds the ending threshold, at which point it is determined that the current EEG sample value has reached the end point.
Step 504: measuring the next EEG sample value

The next EEG sample value is measured, the purpose of which is to compare this next EEG sample value with the ending threshold obtained in the previous step.
Step 505: comparing the next EEG sample value with the ending threshold

When comparing the next EEG sample value with the ending threshold, if the next EEG sample value exceeds the ending threshold, then the initial EEG sample value of the next EEG sample value is the end EEG sample value of the current EEG sample value.

Step 506: if the next EEG sample value exceeds the ending threshold, then the amplitude of the half-wave digital signal is calculated, where the amplitude is the difference between the end EEG sample value of the current EEG sample value and the initial EEG sample value of the current EEG sample value; otherwise, return to continue to perform step 504: continuing to measure the next EEG sample value.

Through the above technical solution, the self-responsive detection parameter optimization method for implantable electrical stimulation devices provided by this application first collects sample signals based on the current detection parameters, and after determining that the sample signals exceed the preset detection threshold, self-responsively obtains the optimal detection parameters. The detection threshold here is a range value of the electroencephalogram (EEG) signal when determining whether conditions such as epilepsy are occurring or about to occur based on the collected sample signal, for example, the EEG signal. If the current detection parameter is set too small, it may trigger responsive treatment under normal circumstances, leading to too high sensitivity of the responsive treatment, and high sensitivity also increases the risk of side effects. Therefore, after determining that the sample signal exceeds the preset detection threshold, it self-responsively obtains the optimal detection parameters, and adjusts the current detection parameters based on the obtained optimal detection parameters, that is, it adjusts the current detection parameters to the optimal detection parameters. In this way, further optimization can be made to the current detection parameters, so that during the real-time detection process of the patient, accurate detection of events such as epilepsy can be achieved, while reducing the rate of misdiagnosis, triggering responsive treatment, achieving the purpose of precision treatment, and timely intervening treatment to prevent the occurrence of events such as epilepsy.

### Embodiment 2

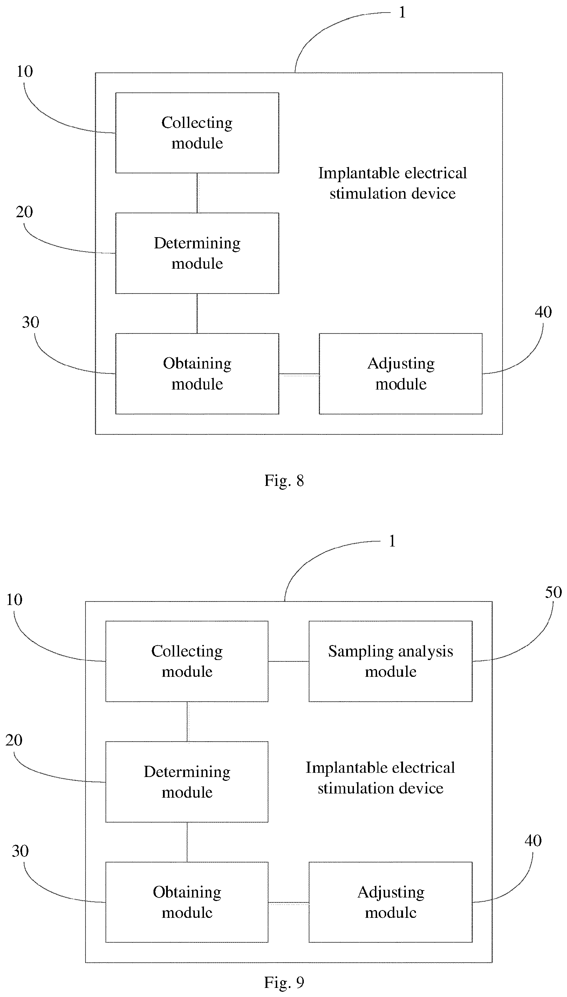

Referring to Fig. 8, it shows an implantable electrical stimulation device 1 provided in the embodiments of this specification. This implantable electrical stimulation device is suitable for the clinical medical implantable electrical stimulation system, which can perform real-time detection on patients such as epilepsy, and perform precise detection on events such as epilepsy during the treatment process, so that early intervention and treatment can be done to prevent the occurrence of events such as epilepsy. This implantable electrical stimulation device continuously obtains the optimal detection parameters during the real-time detection process, so that more accurate sample signals can be collected based on this optimal detection parameter. This implantable electrical stimulation device comprises:
A collecting module 10, which collects sample signals based on current detection parameters.

The first thing an implantable electrical stimulation device needs to do is to collect sample signals, such as the patient's intracranial electroencephalographic signals, to get EEG sample values. Sample signals can also be electrocardiographic signals, nerve electrical signals, etc., depending on the implantable electrical stimulation device. The current detection parameters can be initially set by medical personnel after making a determination based on the patient's condition. The current detection parameters generally include detection algorithms, detection thresholds, detection intervals, etc., and the factory default values of the detection equipment can also be used for the current detection parameters.

A determining module 20, which determines that the sample signal exceeds the preset detection threshold.

After obtaining the sample signals, they are processed and compared with the preset detection threshold to determine whether to optimize the current detection parameters. The setting principle of the preset detection threshold can be determined according to the detection accuracy and sensitivity of events such as epilepsy. The preset detection threshold can be set by medical personnel, such as according to the parameters contained in the sample signal. When the sample signal contains multiple parameters, the corresponding preset detection thresholds can be numerous. When the sample signal exceeds one of the preset detection thresholds, then it is determined that the sample signal exceeds the preset detection threshold. For example, the preset detection threshold can be 50% of the sample signal when events such as epilepsy occur.

An obtaining module 30, which self-responsively obtains the optimal detection parameters

After determining that the sample signal exceeds the preset detection threshold, the optimal detection parameters are self-responsively obtained without any manual activation. The goal here is to replace the current detection parameters with the optimal ones.

The optimal detection parameters can be detection parameters that have been preset based on clinical experience, or they can be detection parameters obtained in other ways; no specific limitation is provided here.

An adjusting module 40, which adjusts the current detection parameters based on the optimal detection parameters.

Based on the obtained optimal detection parameters, the microprocessor of the implantable electrical stimulation device can send the parameter adjustment instructions and the optimal detection parameters to the sampling circuit. After receiving the parameter adjustment instructions, the sampling circuit executes them. The optimal detection parameters replace the current ones and become the detection parameters for the collecting of the next sample signal.

In some embodiments, the implantable electrical stimulation device provided in this embodiment comprises an obtaining module 30 specifically for:
Step 300: self-responsively calculating the current event detection rate

The self-responsive obtaining of optimal detection parameters in Step 30 can be obtained through the self-responsive detection parameter optimization method provided in the embodiments of this specification. After determining that the sample signal exceeds the preset detection threshold, it is determined that events such as epilepsy have been detected. Next, the current event detection rate is calculated self-responsively. The current event detection rate is the detection rate of events like epilepsy up to the present. The calculation of the detection rate can be the number of detected events like epilepsy from the start of the detection to the present, divided by the total number of detections. The detection can be performed at set intervals during real-time detection, so the total number of detections is known. The number of detected events like epilepsy is also known, so the current event detection rate can be calculated.
Step 310: determining that the current event detection rate exceeds the preset target detection rate

The preset target detection rate can be set based on actual clinical experience, for example, the preset target detection rate is 80% for a week. Whether the current event detection rate exceeds the preset target detection rate is determined. After determining that the current event detection rate is greater than the preset target detection rate, it can be inferred that the current detection parameters for events like epilepsy are not precise and sensitive enough, and the current detection parameters need to be further optimized to detect events like epilepsy more timely and accurately.
Step 320: self-responsively calculating the optimal detection parameters

The optimal detection parameters are self-responsively calculated. After obtaining the optimal detection parameters, the implantable electrical stimulation device sends the optimal detection parameters and the parameter adjustment instructions to the collecting circuit. The collecting circuit adjusts the original current detection parameters based on the optimal detection parameters.

Referring to Fig. 9, in some embodiments, the implantable electrical stimulation device provided in the embodiments of this specification also comprises a sample analysis module 50. The sample analysis module 50 is used for:
Step 50: setting the sample analysis result as the detection flag

Before each detection cycle begins to collect sample signals, the sample analysis result can be set as a detection flag. The sample analysis result here can be a recent sample analysis result of the patient as current history. After analyzing the recent sample analysis result, the sample analysis result obtained can be used to determine the symptoms or physiological characteristics of the patient when events like epilepsy occur. These can be quantified as waveform features, and then the sample analysis result can be set as a detection flag. The sample analysis results include half-wave features such as amplitude and duration. Subsequent detections are based on the detection flag. For example, different current detection parameters are used to collect sample signals. Different patients have different recent sample analysis results at different times, corresponding detection flags can be set to carry out corresponding detections, which improves the individualization and targeting of real-time detection of patients.

In some embodiments, the implantable electrical stimulation device provided in the embodiments of this specification comprises a sampling analysis module 50 specifically for:
Step 500: identifying the waveform characteristics of the sample signal

The waveform characteristics of the sample signal can be identified by observing the changes in the amplitude of the sample signal, such as identifying the half-wave features of the sample signal, which can be used to distinguish between epileptic waves and normal brain waves. The sample signal is a recent sample analysis result, which can be the sample signal during the last sampling window.

The waveform characteristics of the sample signal can be identified. If the waveform characteristics match the symptoms or physiological characteristics of the patient's epileptic event, then these waveform characteristics are marked and stored for use as detection flags during the upcoming sampling window.
Step 510: calculating the window parameters of the sample signal during the sampling window

The sampling window period is the time during which real-time detection of the patient is carried out. Calculation of the window parameters of the sample signal during the last sampling window, such as changes in sampling amplitude and frequency, can include calculation of the line length function and area function of that sampling window period. The window parameters of the sample signal during the last sampling window can be used as detection flags for the upcoming sampling window.
Step 520: using the waveform characteristics and window parameters as sample analysis results, and determining whether the sample analysis results match the characteristics of event occurrence

The collected sample signal is compared with the preset detection threshold, and the current detection parameters can be automatically adjusted. Before this, the collected electrical signals are subjected to feature identification. As waveform features themselves have individual differences and even complexity, different features identified can be combined in different ways, such as the combination of line length and area, and the combination of line length and bandpass, etc., to more accurately detect and identify a wider variety of features to improve the accuracy of real-time detection and prediction, and increase the detection rate of brain electrical signals during the occurrence of events such as epilepsy, and prevent the occurrence of epileptic events.

After obtaining the waveform characteristics, such as half-wave features, and window parameters as recent sample analysis results separately, whether the waveform characteristics and window parameters match the symptoms and physiological characteristics of event occurrence is further determined. If they match, then these sample analysis results are set as the detection flag. After the detection flag is set, corresponding detection operations are performed based on the detection flag.

Step 530: if so, setting the sample analysis result as the detection flag.

It is determined whether the waveform characteristics and window parameters of the sample signal during the recent sampling window period as the sample analysis results are the physiological characteristics of the patient's epileptic seizure. If so, the sample analysis results are set as the detection flag, and then the corresponding detection instructions are enabled, so that the corresponding detection parameters can be used to collect sample signals.

In some embodiments, in the implantable electrical stimulation device provided in the embodiments of this specification, the sampling analysis module 50 is further specifically used for:
Step 501: identifying the half-wave digital signal of the sample signal. The half-wave digital signal comprises a starting point and an ending point. The starting point is the initial EEG sample value of the EEG sample values, and the ending point is the ending EEG sample value of the EEG sample values.

The identification of the waveform characteristic of the sample signal can be the identification of the half-wave digital signal of the sample signal. The half-wave digital signal comprises the starting point and ending point. The sample signal is the sample signal within a sampling window period, and the corresponding starting point is the initial EEG sample value of the recent EEG sample value within this sampling window period, and the ending point is the final sample value of the EEG sample value during this sampling window period.

Step 502: calculating the half-wave duration of the half-wave digital signal. The half-wave duration is the difference between the time point corresponding to the ending point and the time point corresponding to the starting point.

The half-wave duration of the half-wave digital signal can be calculated. This half-wave duration is the difference between the time point corresponding to the ending point and the time point corresponding to the starting point.

In some embodiments, in the implantable electrical stimulation device provided in the embodiments of the present specification, the sampling analysis module 50, is also specifically used for:
Step 503: calculating the ending threshold based on the current EEG sample value and the preset hysteresis value

The current EEG sample value mentioned here is the EEG sample value within the current sampling window period. The preset hysteresis value is preset. The hysteresis value refers to the hysteresis value of the sample value. The preset hysteresis value is a fixed value and does not change with each sampling window period. The ending threshold is used to compare with the next EEG sample value to determine the end EEG sample value of the current EEG sample value.

The ending threshold is calculated based on the EEG sample value during the recent sampling window period and the preset hysteresis value. The ending threshold is a range value obtained by adding or subtracting the hysteresis value from the initial EEG sample value of the EEG sample value. Then, the current EEG sample value within the current sampling window period is measured in sequence, the current EEG sample value with the ending threshold is compared; if the current EEG sample value is less than the ending threshold, the ending threshold is recalculated, the current EEG sample value is measured, the current EEG sample value is compared with the ending threshold, until the current EEG sample value exceeds the ending threshold, indicating that the current EEG collection value has reached the end point.
Step 504: measuring the next EEG sample value

The next EEG sample value is measured. The purpose is to compare this next EEG sample value with the ending threshold obtained in the previous step.
Step 505: comparing the next EEG sample value with the ending threshold

When comparing the next EEG sample value with the ending threshold, if the next EEG sample value exceeds the ending threshold, then the initial EEG sample value of the next EEG sample value is the final EEG sample value of the current EEG sample value.

Step 506: if the next EEG sample value exceeds the ending threshold, the amplitude of the half-wave digital signal is calculated. The amplitude is the difference between the final EEG sample value of the current EEG sample value and the initial EEG sample value of the current EEG sample value; otherwise, return to continue executing Step 504: continuing to measure the next EEG sample value.

Through the above technical scheme, the self-responsive detection parameter optimization method for the implantable electric stimulation device provided by this application first collects sample signals based on the current detection parameters, and after determining that the sample signal exceeds the preset detection threshold, it self-responsively obtains the optimal detection parameters. The detection threshold here is a range value of the EEG signal when determining whether conditions such as epilepsy are occurring or about to occur based on the collected sample signal, for example, the EEG signal. If the current detection parameters are set too small, it is possible to trigger a responsive treatment under normal circumstances, leading to overly high sensitivity of the responsive treatment. High sensitivity also increases the risk of side effects. Therefore, after determining that the sample signal exceeds the preset detection threshold, the optimal detection parameters are self-responsively obtained, and the current detection parameters are adjusted based on the obtained optimal detection parameters, that is, adjusting the current detection parameters to the optimal detection parameters. In this way, the current detection parameters can be further optimized, so that precise detection of events such as epilepsy can be carried out in the process of real-time detection of the patient, reducing the false positive rate, triggering responsive treatment, achieving the purpose of precise treatment, and timely intervening in treatment to prevent the occurrence of events such as epilepsy.

### Embodiment 3

This embodiment provided in this specification offers an implantable electrical stimulation system, which comprises an external device and an implantable electrical stimulation device as shown in Figures 8 and 9, with communication between the external device and the implantable electrical stimulation device. This implantable electrical stimulation device is applicable in clinical medicine's implantable electrical stimulation systems, capable of real-time monitoring of patients such as those with epilepsy, and providing precise predictions of epileptic events during treatment, thus allowing early intervention and treatment to avoid the occurrence of events such as epilepsy. The implantable electrical stimulation device continuously obtains the optimal detection parameters during real-time detection, thus collecting more accurate sample signals based on the optimal detection parameters. The implantable electrical stimulation device comprises:
A collecting module 10, which collects sample signals based on the current detection parameters.

The first thing the implantable electrical stimulation device needs to do is to collect sample signals, such as intracranial EEG signals from the patient, to get EEG sample values. The sample signals can also be electrocardiograph (ECG) signals, neural signals, etc., depending on the implantable electrical stimulation device. The current detection parameters could be initially set by medical staff based on their determination of the patient's condition. The current detection parameters generally include detection algorithms, detection thresholds, detection intervals, etc., and the factory default values of the detection device can also be used as the current detection parameters.

A determining module 20, which determines if the sample signal exceeds the preset detection threshold.

After obtaining the sample signals, the device processes them, comparing them with the preset detection threshold, to decide whether to optimize the current detection parameters. The setting principle of the preset detection threshold can be determined according to the detection accuracy and sensitivity of events such as epilepsy. The preset detection threshold can be set by medical staff, for example, based on the parameters contained in the sample signal. When the sample signal contains multiple parameters, the corresponding preset detection threshold can include multiple parameters. When the sample signal contains multiple parameters, the corresponding preset detection thresholds can be numerous. When the sample signal exceeds one of the preset detection thresholds, then it is determined that the sample signal exceeds the preset detection threshold. For example, the preset detection threshold can be 50% of the sample signal when events such as epilepsy occur.

An obtaining module 30, which self-responsively obtains optimal detection parameters.

After determining that the sample signal exceeds the preset detection threshold, the system self-responsively obtains the optimal detection parameters, with the aim of replacing the current detection parameters with the optimal ones.

The optimal detection parameters could be those previously set based on clinical experience, or they could be obtained in other ways; no specific limitation is provided here.

An adjusting module 40, which adjusts the current detection parameters based on the optimal detection parameters.

Based on the obtained optimal detection parameters, the current detection parameters are adjusted. The microprocessor of the implantable electrical stimulation device can send parameter adjustment instructions and optimal detection parameters to the sampling circuit. After receiving the parameter adjustment instruction, the sampling circuit executes the parameter adjustment instruction, replacing the current detection parameters with the optimal ones to become the next set of detection parameters for collecting sample signals.

With this technical solution, the self-responsive detection parameter optimization method provided by this application for the implantable electrical stimulation device first collects sample signals based on the current detection parameters. Once it determines that the sample signal exceeds the preset detection threshold, it self-responsively obtains the optimal detection parameters. The detection threshold here is a range value of the EEG signal when determining whether conditions such as epilepsy are occurring or about to occur based on the collected sample signal, for example, the EEG signal. If the current detection parameters are set too small, it is possible to trigger a responsive treatment under normal circumstances, leading to overly high sensitivity of the responsive treatment. High sensitivity also increases the risk of side effects. Therefore, after determining that the sample signal exceeds the preset detection threshold, the optimal detection parameters are self-responsively obtained, and the current detection parameters are adjusted based on the obtained optimal detection parameters, that is, adjusting the current detection parameters to the optimal detection parameters. In this way, the current detection parameters can be further optimized, so that precise detection of events such as epilepsy can be carried out in the process of real-time detection of the patient, reducing the false positive rate, triggering responsive treatment, achieving the purpose of precise treatment, and timely intervening in treatment to prevent the occurrence of events such as epilepsy.

### Embodiment 4

This specification provides a storage medium for computer-readable storage. The storage medium stores one or more programs. When executed by one or more processors, the steps of the self-responsive detection parameter optimization method shown in Figures 1 to 7 are implemented, specifically comprising:
Step 10: collecting sample signals based on the current detection parameters

The first thing the implantable electrical stimulation device needs to do is to collect sample signals, such as intracranial EEG signals from the patient, to get EEG sample values. The sample signals can also be ECG signals, neural signals, etc., depending on the implantable electrical stimulation device. The current detection parameters could be initially set by medical staff based on their determination of the patient's condition. The current detection parameters generally include detection algorithms, detection thresholds, detection intervals, etc., and the factory default values of the detection device can also be used as the current detection parameters.
Step 20: Determining that the sample signal exceeds the preset detection threshold;

After obtaining the sample signals, the device processes them, comparing them with the preset detection threshold, to decide whether to optimize the current detection parameters. The setting principle of the preset detection threshold can be determined according to the detection accuracy and sensitivity of events such as epilepsy. The preset detection threshold can be set by medical staff, for example, based on the parameters contained in the sample signal. When the sample signal contains multiple parameters, the corresponding preset detection threshold can include multiple parameters. When the sample signal contains multiple parameters, the corresponding preset detection thresholds can be numerous. When the sample signal exceeds one of the preset detection thresholds, then it is determined that the sample signal exceeds the preset detection threshold. For example, the preset detection threshold can be 50% of the sample signal when events such as epilepsy occur.
Step 30: self-responsively obtaining optimal detection parameters

After determining that the sample signal exceeds the preset detection threshold, the optimal detection parameters are self-responsively obtained, with the aim of replacing the current detection parameters with the optimal ones.

The optimal detection parameters can be detection parameters that have been preset based on clinical experience, or they can be detection parameters obtained in other ways; no specific limitation is provided here.
Step 40: adjusting the current detection parameters based on the optimal detection parameters

Based on the obtained optimal detection parameters, the current detection parameters are adjusted. The microprocessor of the implantable electrical stimulation device can send a parameter adjustment instruction and the optimal detection parameters to the sampling circuit. After receiving the parameter adjustment instruction, the sampling circuit executes the parameter adjustment instruction, replacing the current detection parameters with the optimal ones to become the next set of detection parameters for collecting sample signals.

Through the above technical solution, the self-responsive detection parameter optimization method for implantable electrical stimulation devices provided by this application first collects sample signals based on the current detection parameters, and after determining that the sample signals exceed the preset detection threshold, self-responsively obtains the optimal detection parameters. The detection threshold here is a range value of the electroencephalogram (EEG) signal when determining whether conditions such as epilepsy are occurring or about to occur based on the collected sample signal, for example, the EEG signal. If the current detection parameter is set too small, it may trigger responsive treatment under normal circumstances, leading to too high sensitivity of the responsive treatment, and high sensitivity also increases the risk of side effects. Therefore, after determining that the sample signal exceeds the preset detection threshold, it self-responsively obtains the optimal detection parameters, and adjusts the current detection parameters based on the obtained optimal detection parameters, that is, it adjusts the current detection parameters to the optimal detection parameters. In this way, further optimization can be made to the current detection parameters, so that during the real-time detection process of the patient, accurate detection of events such as epilepsy can be achieved, while reducing the rate of misdiagnosis, triggering responsive treatment, achieving the purpose of precision treatment, and timely intervening treatment to prevent the occurrence of events such as epilepsy.

In conclusion, the above description is only the preferred embodiment of this specification and is not intended to limit the scope of protection of this specification. Any modifications, equivalent replacements, improvements, etc., made within the spirit and principle of this specification should be included within the scope of protection of this specification.

The system, device, module, or unit elaborated in the above one or more embodiments can be specifically implemented by computer chips or entities, or by products with certain functions. A typical implementation device is a computer. Specifically, computers can include personal computers, laptops, cell phones, camera phones, smartphones, personal digital assistants, media players, navigation devices, email devices, game consoles, tablet computers, wearable devices, or a combination of any of these devices.

Computer-readable storage media include both permanent and non-permanent, movable and immovable media implemented by any method or technology for information storage. Information can be computer-readable instructions, data structures, program modules, or other data. Examples of computer storage media include, but are not limited to, phase-change memory (PRAM), static random-access memory (SRAM), dynamic random-access memory (DRAM), other types of random-access memory (RAM), read-only memory (ROM), electrically erasable programmable read-only memory (EEPROM), flash memory or other memory technologies, compact disc read-only memory (CD-ROM), digital versatile discs (DVD) or other optical storage, magnetic tape, magnetic disk storage or other magnetic storage devices, or any other non-transitory medium that can be used to store information accessible by a computing device. According to the definition in this disclosure, computer-readable media do not include transitory computer-readable media, such as modulated data signals and carriers.

It should also be noted that the terms "comprising," "including," or any other variation thereof, are intended to cover a non-exclusive inclusion, such that a process, method, article, or device that includes a list of elements not only includes those elements, but also includes other elements not explicitly listed, or further includes inherent elements of such a process, method, article, or device. Without further constraints, an element defined by the statement "comprising an..." does not preclude the presence of other identical elements in the process, method, article, or device that includes the stated element.

The various embodiments in this specification are described in a progressive manner. The same or similar parts between each embodiment can be referred to each other, and what is emphasized in each embodiment is the differences from other embodiments. In particular, for system embodiments, since they are basically similar to method embodiments, the description is relatively simple, and the related parts can be referred to the description of the method embodiments.

The above description has been made for specific embodiments of this specification. Other embodiments are within the scope of the appended claims. In some cases, the actions or steps recorded in the claims can be executed in a different order from the embodiments and still achieve the expected results. Additionally, the processes depicted in the figures do not necessarily require a specific order or sequential order to achieve the desired results. In some embodiments, multitasking and parallel processing can also be possible or advantageous. The present invention is set out in the claims that follow.

## Claims

1. A self-responsive detection parameter optimization method for an implantable electrical stimulation device, comprising:
identifying (S500) waveform characteristics of a sample signal;
calculating (S510) window parameters of the sample signal during a sampling window period;
using (S520) the waveform characteristics and the window parameters as sample analysis results, and determining whether the sample analysis results meet characteristics of an event occurrence;
if so, setting (S530) the sample analysis results as a detection flag associated with a current physiological state of a patient;
selecting, from a plurality of pre-stored detection parameter sets each corresponding to a different detection flag, a current detection parameter set corresponding to the set detection flag;
collecting (S100) sample signals using the selected current detection parameter set;
determining (S20) that a collected sample signal exceeds a preset detection threshold;
self-responsively calculating (S30) optimal detection parameters in response to said determining, wherein the calculation is based on a comparison between a current event detection rate and a preset target detection rate;
adjusting (S40) the current detection parameter set based on the calculated optimal detection parameters to form a closed-loop parameter optimization system, wherein the adjusted parameters are applied in a subsequent sampling window period.

2. The self-responsive detection parameter optimization method as claimed in claim 1, wherein identifying the waveform characteristics of the sample signal specifically comprises:
identifying (S501) a half-wave digital signal of the sample signal, the half-wave digital signal including a starting point and an ending point, wherein the starting point is an initial EEG sample value of EEG sample values, and the ending point is a final EEG sample value of the EEG sample values;
calculating (S502) a duration of the half-wave digital signal, the duration of the half-wave is a difference between a time point corresponding to the ending point and a time point corresponding to the starting point.

3. The self-responsive detection parameter optimization method as claimed in claim 2, wherein identifying the half-wave digital signal of the sample signal specifically comprises:
calculating (S503) an ending threshold based on a current EEG sample value and a preset hysteresis value;
measuring (S504) a next EEG sample value;
comparing (S505) the next EEG sample value with the ending threshold;
if the next EEG sample value exceeds the ending threshold, setting an initial EEG sample value of the next EEG sample value as the final EEG sample value of the current EEG sample value;
calculating (S506) an amplitude of the half-wave digital signal, wherein the amplitude is a difference between the final EEG sample value and the initial EEG sample value.

4. An implantable electrical stimulation device, comprising:
a sample analysis module (50) for:
identifying waveform characteristics of a sample signal;
calculating window parameters of the sample signal during a sampling window period;
using the waveform characteristics and the window parameters as sample analysis results, and
determining whether the sample analysis results meet characteristics of an event occurrence;
if so, setting the sample analysis results as a detection flag associated with a current physiological state of a patient;
a parameter selection module (60) for selecting, from a plurality of pre-stored detection parameter sets each corresponding to a different detection flag, a current detection parameter set corresponding to the detection flag set by the sample analysis module;
a collecting module (10) for collecting sample signals using the current detection parameter set selected by the parameter selection module;
a determining module (20) for determining that a collected sample signal exceeds a preset detection threshold;
an optimal parameter calculation module (30) for, in response to said determination, self-responsively calculating optimal detection parameters based on a comparison between a current event detection rate and a preset target detection rate;
an parameter adjusting module (40) for adjusting the current detection parameter set based on the calculated optimal detection parameters to form a closed-loop parameter optimization system, wherein the adjusted parameters are applied in a subsequent sampling window period.

5. The implantable electrical stimulation device as claimed in claim 4, wherein the sample analysis module (50) is for:
identifying a half-wave digital signal of the sample signal, the half-wave digital signal including a starting point and an ending point, wherein the starting point is an initial EEG sample value of EEG sample values, and the ending point is a final EEG sample value of the EEG sample values;
calculating a duration of the half-wave digital signal, wherein the duration of the half-wave is a difference between a time point corresponding to the ending point and a time point corresponding to the starting point.

6. The implantable electrical stimulation device as claimed in claim 5, wherein the sample analysis module (50) is for:
calculating an ending threshold based on a current EEG sample value and a preset hysteresis value;
measuring a next EEG sample value;
comparing the next EEG sample value with the ending threshold;
if the next EEG sample value exceeds the ending threshold, setting an initial EEG sample value of the next EEG sample value as the final EEG sample value of the current EEG sample value;
calculating an amplitude of the half-wave digital signal, wherein the amplitude is a difference between the final EEG sample value and the initial EEG sample value.

7. An implantable electrical stimulation system, comprising an external device and the implantable electrical stimulation device as claimed in any one of claims 4 to 6, wherein the external device is communicatively connected with the implantable electrical stimulation device.

8. A computer-readable storage medium, the storage medium storing one or more programs, when the one or more programs are executed by one or more processors, the steps of the self-responsive detection parameter optimization method as claimed in any one of claims 1 to 3 are implemented; wherein the collecting of sample signals is implemented by the collecting module of the implantable electrical stimulation device.

## Patentansprüche

1. Ein Verfahren zur selbstreagierenden Optimierung von Detektionsparametern für eine implantierbare elektrische Stimulationsvorrichtung, umfassend:
Identifizieren (S500) von Wellenformmerkmalen eines Probensignals;
Berechnen (S510) von Fensterparametern des Probensignals während einer Abtastfensterperiode;
Verwenden (S520) der Wellenformmerkmale und der Fensterparameter als Probenanalyseergebnisse, und Bestimmen, ob die Probenanalyseergebnisse Merkmale eines Ereignisauftretens erfüllen;
falls ja, Setzen (S530) der Probenanalyseergebnisse als Detektionsflag, das einem aktuellen physiologischen Zustand eines Patienten zugeordnet ist;
Auswählen eines aktuellen Detektionsparametersatzes, der dem gesetzten Detektionsflag entspricht, aus einer Vielzahl von vorab gespeicherten Detektionsparametersätzen, die jeweils einem unterschiedlichen Detektionsflag entsprechen;
Sammeln (S100) von Probensignalen unter Verwendung des ausgewählten aktuellen Detektionsparametersatzes;
Bestimmen (S20), dass ein gesammeltes Probensignal einen voreingestellten Detektionsschwellenwert überschreitet;
selbstreagierendes Berechnen (S30) optimaler Detektionsparameter als Reaktion auf das genannte Bestimmen, wobei das Berechnen auf einem Vergleich zwischen einer aktuellen Ereignisdetektionsrate und einer voreingestellten Zieldetektionsrate basiert;
Einstellen (S40) des aktuellen Detektionsparametersatzes basierend auf den berechneten optimalen Detektionsparametern, um ein Parameteroptimierungssystem von geschlossenem Kreis zu bilden, wobei die eingestellten Parameter in einer nachfolgenden Abtastfensterperiode angewendet werden.

2. Das Verfahren zur selbstreagierenden Optimierung von Detektionsparametern nach Anspruch 1, wobei das Identifizieren von den Wellenformmerkmalen des Probensignals konkret umfasst:
Identifizieren (S501) eines digitalen Halbwellensignals des Probensignals, wobei das digitale Halbwellensignal einen Startpunkt und einen Endpunkt umfasst, wobei der Startpunkt ein anfänglicher EEG-Probenwert von EEG-Probenwerten ist und der Endpunkt ein letzter EEG-Probenwert von den EEG-Probenwerten ist;
Berechnen (S502) einer Dauer des digitalen Halbwellensignals, wobei die Dauer der Halbwelle eine Differenz zwischen einem dem Endpunkt entsprechenden Zeitpunkt und einem dem Startpunkt entsprechenden Zeitpunkt ist.

3. Das Verfahren zur selbstreagierenden Optimierung von Detektionsparametern nach Anspruch 2, wobei das Identifizieren des digitalen Halbwellensignals des Probensignals konkret umfasst:
Berechnen (S503) eines Endschwellenwerts basierend auf einem aktuellen EEG-Probenwert und einem voreingestellten Hysterese-Wert;
Messen (S504) eines nächsten EEG-Probenwerts;
Vergleichen (S505) des nächsten EEG-Probenwerts mit dem Endschwellenwert;
falls der nächste EEG-Probenwert den Endschwellenwert überschreitet, Setzen eines anfänglichen EEG-Probenwerts des nächsten EEG-Probenwerts als den letzten EEG-Probenwert des aktuellen EEG-Probenwerts;
Berechnen (S506) einer Amplitude des digitalen Halbwellensignals, wobei die Amplitude eine Differenz zwischen dem letzten EEG-Probenwert und dem anfänglichen EEG-Probenwert ist.

4. Eine implantierbare elektrische Stimulationsvorrichtung, umfassend:
ein Probenanalysemodul (50) zum:
Identifizieren von Wellenformmerkmalen eines Probensignals;
Berechnen von Fensterparametern des Probensignals während einer Abtastfensterperiode;
Verwenden der Wellenformmerkmale und der Fensterparameter als Probenanalyseergebnisse; und
Bestimmen, ob die Probenanalyseergebnisse Merkmale eines Ereignisauftretens erfüllen;
falls ja, Setzen der Probenanalyseergebnisse als Detektionsflag, das einem aktuellen physiologischen Zustand eines Patienten zugeordnet ist;
ein Parameterauswählungsmodul (60) zum Auswählen eines aktuellen Detektionsparametersatzes, der dem durch das Probenanalysemodul gesetzten Detektionsflag entspricht, aus einer Vielzahl von vorab gespeicherten Detektionsparametersätzen, die jeweils einem unterschiedlichen Detektionsflag entsprechen;
ein Sammlungsmodul (10) zum Sammeln von Probensignalen unter Verwendung des durch das Parameterauswählungsmodul ausgewählten aktuellen Detektionsparametersatzes;
ein Bestimmungsmodul (20) zum Bestimmen, dass ein gesammeltes Probensignal einen voreingestellten Detektionsschwellenwert überschreitet;
ein Modul (30) zur Berechnung optimaler Parameter für selbstreagierende Berechnung, als Reaktion auf das genannte Bestimmen, optimaler Detektionsparameter basierend auf einem Vergleich zwischen einer aktuellen Ereignisdetektionsrate und einer voreingestellten Zieldetektionsrate;
ein Parametereinstellungsmodul (40) zum Einstellen des aktuellen Detektionsparametersatzes basierend auf den berechneten optimalen Detektionsparametern, um ein Parameteroptimierungssystem von geschlossenem Kreis zu bilden, wobei die eingestellten Parameter in einer nachfolgenden Abtastfensterperiode angewendet sind.

5. Die implantierbare elektrische Stimulationsvorrichtung nach Anspruch 4, wobei das Probenanalysemodul (50) dazu dient:
ein digitales Halbwellensignal des Probensignals zu identifizieren, wobei das digitale Halbwellensignal einen Startpunkt und einen Endpunkt umfasst, wobei der Startpunkt ein anfänglicher EEG-Probenwert von EEG-Probenwerten ist und der Endpunkt ein letzter EEG-Probenwert von den EEG-Probenwerten ist;
eine Dauer des digitalen Halbwellensignals zu berechnen, wobei die Dauer der Halbwelle eine Differenz zwischen einem dem Endpunkt entsprechenden Zeitpunkt und einem dem Startpunkt entsprechenden Zeitpunkt ist.

6. Die implantierbare elektrische Stimulationsvorrichtung nach Anspruch 5, wobei das Probenanalysemodul (50) dazu dient:
einen Endschwellenwert basierend auf einem aktuellen EEG-Probenwert und einem voreingestellten Hysterese-Wert zu berechnen;
ein nächsten EEG-Probenwert zu messen;
den nächsten EEG-Probenwert mit dem Endschwellenwert zu vergleichen;
falls der nächste EEG-Probenwert den Endschwellenwert überschreitet, einen anfänglichen EEG-Probenwert des nächsten EEG-Probenwerts als den letzen EEG-Probenwert des aktuellen EEG-Probenwerts zu setzen;
Berechnen eine Amplitude des digitalen Halbwellensignals, wobei die Amplitude eine Differenz zwischen dem letzen EEG-Probenwert und dem anfänglichen EEG-Probenwert ist.

7. Ein Implantierbares elektrisches Stimulationssystem, umfassend eine externe Vorrichtung und die implantierbare elektrische Stimulationsvorrichtung nach einem der Ansprüche 4 bis 6, wobei die externe Vorrichtung kommunikativ mit der implantierbaren elektrischen Stimulationsvorrichtung verbunden ist.

8. Ein computerlesbares Speichermedium, wobei das Speichermedium ein oder mehrere Programme speichert, wenn das eine oder die mehreren Programme durch einen oder mehrere Prozessoren ausgeführt werden, die Schritte des Verfahrens zur selbstreagierenden Optimierung von Detektionsparametern nach einem der Ansprüche 1 bis 3 implementiert werden; wobei das Sammeln von Probensignalen durch das Sammelungsmodul der implantierbaren elektrischen Stimulationsvorrichtung implementiert wird.

## Revendications

1. Procédé d'optimisation de paramètres de détection en auto-réponse pour un dispositif de stimulation électrique implantable, comprenant:
l'identification (S500) de caractéristiques de forme d'onde d'un signal échantillon;
le calcul (S510) de paramètres de fenêtre du signal échantillon pendant une période de fenêtre d'échantillonnage;
l'utilisation (S520) des caractéristiques de forme d'onde et des paramètres de fenêtre comme résultats d'analyse d'échantillon, et la détermination si les résultats d'analyse d'échantillon satisfont aux caractéristiques d'une occurrence d'événement;
si tel est le cas, la définition (S530) des résultats d'analyse d'échantillon comme un signe de détection associé à un état physiologique courant d'un patient;
la sélection, parmi une pluralité d'ensembles de paramètres de détection pré-stockés correspondant chacun à un signe de détection différent, d'un ensemble courant de paramètres de détection correspondant au signe de détection défini;
la collection (S100) de signaux échantillons en utilisant l'ensemble courant de paramètres de détection sélectionné;
la détermination (S20) qu'un signal échantillon collecté dépasse un seuil de détection prédéfini;
le calcul (S30) en auto-réponse de paramètres de détection optimaux en réponse à ladite détermination, le calcul étant basé sur une comparaison entre un taux de détection d'événement courant et un taux de détection cible prédéfini;
l'ajustement (S40) de l'ensemble courant de paramètres de détection sur la base des paramètres de détection optimaux calculés afin de former un système d'optimisation de paramètres en boucle fermée, les paramètres ajustés étant appliqués dans une période de fenêtre d'échantillonnage ultérieure.

2. Procédé d'optimisation de paramètres de détection en auto-réponse selon la revendication 1, dans lequel l'identification des caractéristiques de forme d'onde du signal échantillon comprend spécifiquement:
l'identification (S501) d'un signal numérique demi-onde du signal échantillon, le signal numérique demi-onde incluant un point de départ et un point de fin, dans lequel le point de départ est une valeur d'échantillon EEG initiale parmi des valeurs d'échantillon EEG, et le point de fin est une valeur d'échantillon EEG finale parmi les valeurs d'échantillon EEG;
le calcul (S502) d'une durée du signal numérique demi-onde, la durée de la demi-onde étant une différence entre un instant correspondant au point de fin et un instant correspondant au point de départ.

3. Procédé d'optimisation de paramètres de détection en auto-réponse selon la revendication 2, dans lequel l'identification du signal numérique demi-onde du signal échantillon comprend spécifiquement:
le calcul (S503) d'un seuil de fin sur la base d'une valeur d'échantillon EEG courante et d'une valeur d'hystérésis prédéfinie;
la mesure (S504) d'une valeur d'échantillon EEG suivante;
la comparaison (S505) de la valeur d'échantillon EEG suivante avec le seuil de fin;
si la valeur d'échantillon EEG suivante dépasse le seuil de fin, la définition d'une valeur d'échantillon EEG initiale de la valeur d'échantillon EEG suivante comme la valeur d'échantillon EEG finale de la valeur d'échantillon EEG courante;
le calcul (S506) d'une amplitude du signal numérique demi-onde, l'amplitude étant une différence entre la valeur d'échantillon EEG finale et la valeur d'échantillon EEG initiale.

4. Dispositif de stimulation électrique implantable, comprenant:
un module d'analyse d'échantillon (50) pour:
identifier des caractéristiques de forme d'onde d'un signal échantillon;
calculer des paramètres de fenêtre du signal échantillon pendant une période de fenêtre d'échantillonnage;
utiliser les caractéristiques de forme d'onde et les paramètres de fenêtre comme résultats d'analyse d'échantillon, et
déterminer si les résultats d'analyse d'échantillon satisfont aux caractéristiques d'une occurrence d'événement;
si tel est le cas, définir les résultats d'analyse d'échantillon comme un signe de détection associé à un état physiologique courant d'un patient;
un module de sélection de paramètres (60) pour sélectionner, parmi une pluralité d'ensembles de paramètres de détection pré-stockés correspondant chacun à un signe de détection différent, un ensemble courant de paramètres de détection correspondant au signe de détection défini par le module d'analyse d'échantillon;
un module de collection (10) pour collecter des signaux échantillons en utilisant l'ensemble courant de paramètres de détection sélectionné par le module de sélection de paramètres;
un module de détermination (20) pour déterminer qu'un signal échantillon collecté dépasse un seuil de détection prédéfini;
un module de calcul de paramètres optimaux (30) pour calculer, en auto-réponse, des paramètres de détection optimaux sur la base d'une comparaison entre un taux de détection d'événement courant et un taux de détection cible prédéfini, en réponse à ladite détermination;
un module d'ajustement de paramètres (40) pour ajuster l'ensemble courant de paramètres de détection sur la base des paramètres de détection optimaux calculés pour former un système d'optimisation de paramètres en boucle fermée, les paramètres ajustés étant appliqués dans une période de fenêtre d'échantillonnage ultérieure.

5. Dispositif de stimulation électrique implantable selon la revendication 4, dans lequel le module d'analyse d'échantillon (50) est destiné à:
identifier un signal numérique demi-onde du signal échantillon, le signal numérique demi-onde incluant un point de départ et un point de fin, dans lequel le point de départ est une valeur d'échantillon EEG initiale parmi des valeurs d'échantillon EEG, et le point de fin est une valeur d'échantillon EEG finale parmi les valeurs d'échantillon EEG;
calculer une durée du signal numérique demi-onde, la durée de la demi-onde étant une différence entre un instant correspondant au point de fin et un instant correspondant au point de départ.

6. Dispositif de stimulation électrique implantable selon la revendication 5, dans lequel le module d'analyse d'échantillon (50) est destiné à:
calculer un seuil de fin sur la base d'une valeur d'échantillon EEG courante et d'une valeur d'hystérésis prédéfinie;
mesurer une valeur d'échantillon EEG suivante;
comparer la valeur d'échantillon EEG suivante avec le seuil de fin;
si la valeur d'échantillon EEG suivante dépasse le seuil de fin, définir une valeur d'échantillon EEG initiale de la valeur d'échantillon EEG suivante comme la valeur d'échantillon EEG finale de la valeur d'échantillon EEG courante;
calculer une amplitude du signal numérique demi-onde, l'amplitude étant une différence entre la valeur d'échantillon EEG finale et la valeur d'échantillon EEG initiale.

7. Système de stimulation électrique implantable, comprenant un dispositif externe et le dispositif de stimulation électrique implantable selon l'une quelconque des revendications 4 à 6, dans lequel le dispositif externe est connecté en communication avec le dispositif de stimulation électrique implantable.

8. Support de stockage lisible par ordinateur, le support de stockage stockant un ou plusieurs programmes, lorsque le ou les programmes sont exécutés par un ou plusieurs processeurs, les étapes du procédé d'optimisation de paramètres de détection en auto-réponse selon l'une quelconque des revendications 1 à 3 sont mises en œuvre; dans lequel la collection de signaux échantillons est mise en œuvre par le module de collection du dispositif de stimulation électrique implantable.
